Europäisches Patentamt

European Patent Office

Office européen des brevets

(19)

(11) Veröffentlichungsnummer: **0 219 762**
**B1**

(12) ## EUROPÄISCHE PATENTSCHRIFT

(45) Veröffentlichungstag der Patentschrift:
27.12.90

(51) Int. Cl.⁵: **A61K 9/24, A61K 9/70**

(21) Anmeldenummer: **86113919.4**

(22) Anmeldetag: **07.10.86**

(54) **Verfahren zur Herstellung einer Darreichungs- und Dosierungsform für Arzneimittel-Wirkstoffe, Reagentien oder andere Wirkstoffe.**

(30) Priorität: **09.10.85 DE 3536024**

(43) Veröffentlichungstag der Anmeldung:
**29.04.87 Patentblatt 87/18**

(45) Bekanntmachung des Hinweises auf die Patenterteilung:
**27.12.90 Patentblatt 90/52**

(84) Benannte Vertragsstaaten:
**AT BE CH DE ES FR GB GR IT LI LU NL SE**

(56) Entgegenhaltungen:
**DE-A- 2 746 414**
**GB-A- 139 077**
**GB-A- 1 061 557**

**CHEMICAL ABSTRACTS, Band 85, Nr. 10, 6. September 1976, Seite 364, Zusammenfassung Nr. 68303m, Columbus, Ohio, US; & JP-A-76 54 917 (TOPPAN PRINTING CO. LTD.) 14.05.1976**

(73) Patentinhaber: **Desitin Arzneimittel GmbH, Weg beim Jäger 214, D-2000 Hamburg 63(DE)**

(72) Erfinder: **Schmidt, Wolfgang, Dr., Reembroden 44, D-2000 Hamburg 63(DE)**

(74) Vertreter: **UEXKÜLL & STOLBERG Patentanwälte, Beselerstrasse 4, D-2000 Hamburg 52(DE)**

## Beschreibung

Arzneimittel können in Form von Pulvern, Tropflösungen oder Säften oral verabreicht werden. Da bei diesen Abgabeformen eine genaue Dosierung jedoch schwierig ist, werden vom Hersteller dosierte Applikationsformen wie Tabletten, Dragees oder Kapseln generell bevorzugt. Auch Reagentien und andere Wirkstoffe, z.B. Süßstoffe oder Aromastoffe, werden für eine genaue dosierte Anwendung häufig tablettiert. Die Herstellungstechnik für Tabletten, Dragees, Kapseln und dergleichen ist zwar weitgehend ausgereift, doch sind eine Reihe von systembedingten Nachteilen nicht zu übersehen.

Für niedrig dosierte Wirkstoffe muß ein großer Anteil an Hilfsstoffen zugesetzt werden, um zu einer handhabbaren Größe der Einzeldosis zu gelangen. Weiterhin ist eine genaue Kennzeichnung einzelner Tabletten oder Dragees praktisch nicht möglich. Es haben sich deshalb Durchdrückpackungen durchgesetzt, welche eine Mehrzahl von Tabletten, Dragees oder auch Kapseln enthalten und welche mit den notwendigen Informationen, insbesondere dem Namen des Präparates bedruckt sind. Die Herstellung solcher Verpackungen erfordert naturgemäß einen zusätzlichen Arbeitsgang und es werden Umverpackungen in Form von Faltschachteln benötigt, welche ein beträchtliches Leervolumen aufweisen und dadurch zusätzlich Lagerraum beanspruchen. Ein besonders gravierender Nachteil von Dragees und Kapseln besteht darin, daß eine Zerteilung praktisch unmöglich ist, die kleinste Dosis somit vorgegeben ist. Auch bei Tabletten ist eine genaue Zerteilung schwierig, lediglich größere Tabletten mit einer Kerbe als Sollbruchstelle lassen sich allenfalls teilen, wobei häufig ungleichgroße Bruchstücke entstehen.

Es sind bereits Versuche zur Schaffung einer neuen Darreichungsform für die orale Verabreichung von Arzneimitteln bekannt geworden, welche aus wirkstoffhaltigen Folien bestehen. Gemäß der BE-A 637 363 wird ein papierartiges Trägermaterial aus unlöslichen Zellulosefasern mit einer Wirkstofflösung getränkt bzw. durch Auftragen oder -streuen beschichtet und eine Dosierung durch Perforation der Trägerfolie nach Art eines Briefmarkenbogens erreicht. Die Wirkstoffdosierung ist dabei zwangsläufig äußerst ungenau. Aus den DE-A 2 432 925 und DE-A 2 449 865 ist es bekannt, Arzneimittelwirkstoffe in Folienbildner einzuarbeiten, bei denen es sich vorzugsweise um wasserlösliche Verbindungen wie Methyl- und Ethylzellulose, insbesondere aber Hydroxypropylzellulose, Hydroxyethylzellulose oder Methylhydroxypropylzellulose handelt. Daneben können die Folien Füllstoffe und Trennmittel enthalten. Die DE-A 2 746 414 beschreibt ebenfalls die Verarbeitung von wirkstoffhaltigen Folienmassen auf Basis von beispielsweise Gelatine oder Zellulosederivaten und weiteren Zusätzen wie Stärke zu Folien, in die der Wirkstoff eingearbeitet ist. Die erhaltenen wirkstoffhaltigen Folien lassen sich zur Dosierung durch Perforation in einzelne Abschnitte aufteilen.

Aus der GB-A 1 061 557 ist es bekannt, Gelatinefolien oder Reispapier mit einer Wirkstofflösung zu imprägnieren oder mit einer Wirkstofflösung bzw. -schmelze zu beschichten. Die Beschichtung erfolgt durch Besprühen mit der Lösung oder durch Laminieren von zwei Trägerfolien mit der dazwischen liegenden Wirkstoffschmelze. Diese Herstellungsverfahren ermöglichen keine exakte Dosierung des Wirkstoffes: Beim Aufsprühen einer Wirkstofflösung kann ebenso wie beim Beschichten mit einer Schmelze eine völlig gleichmäßige Schichtdicke nicht sichergestellt werden. Darüber hinaus haftet die nur aus dem Wirkstoff bestehende Beschichtung häufig schlecht auf der Trägerfolie.

Die JA-A 76/54 917 erwähnt die Möglichkeit, eßbare Folien, z.B. Gelatinefolien, mit Wirkstofflösungen zu bedrucken, welche Verdickungsmittel wie Hydroxylpropylzellulose enthalten. Auch bei dieser Vorgehensweise erhält man häufig nur schlecht haftende Beschichtungen.

Alle diese Vorschläge haben keinen Eingang in die Praxis gefunden und in dem neuesten Lehrbuch der "Arzneiformenlehre" von P.H. List, 4. Auflage, Stuttgart, 1985, finden sie keine Erwähnung. Dies beruht ersichtlich darauf, daß die bislang bekanntgewordenen Vorschläge es nicht ermöglichen, die geforderte Gewichtskonstanz und gleichmäßige Wirkstoffverteilung zu erreichen, welche heute gefordert werden. Die Pharmakopoea Europae setzt zum Beispiel Maßstäbe für die Gleichförmigkeit des Gewichtes einzeldosierter Arzneiformen, wobei diese dem jeweiligen Durchschnittsgewicht entsprechend nach höchstzulässigen Abweichungen in % gestattet sind. Die Forderung liegt im allgemeinen bei +/− 5 bis max. 10%. Entsprechende Werte für feste Arzneiformen bestehen auch hinsichtlich anderer Parameter wie Zerfallzeit und Lösungsgeschwindigkeit.

Die oben erwähnten Vorschläge des Standes der Technik führen zu Produkten ungenügender Akzeptanz durch die Patienten (z.B. lassen sich Papierabschnitte nur schlecht einnehmen) und erlauben keine exakte Dosierung pro Flächeneinheit, wie sie unbedingt gefordert werden muß. Bei Inkorporieren des Wirkstoffes in eine Folie bereitet nicht nur die genaue Dosierung Schwierigkeiten, sondern ein wesentlicher weiterer Nachteil besteht darin, daß für jeden Wirkstoff eine entsprechende Folie gesondert hergestellt werden muß, so daß die Wirtschaftlichkeit des Herstellungsverfahrens nicht gegeben ist.

Der Erfindung liegt demgegenüber die Aufgabe zugrunde, eine "zweidimensionale" Darreichungs- und Dosierungsform zu schaffen, welche die genannten Nachteile nicht aufweist, sich leicht herstellen läßt und mit großer Flexibilität an die Anforderungen des Marktes und verschiedener Wirkstoffe angepaßt werden kann.

Gegenstand der Erfindung ist ein Verfahren zur Herstellung einer Darreichungs- und Dosierungsform für Arzneimittelwirkstoffe, Reagentien oder andere Wirkstoffe in Form einer wasserlöslichen Folie auf Basis von Stärken, Gelatinen, Glycerin und/oder Sorbit sowie gegebenenfalls natürlichen und/oder synthetischen Harzen und Gummen, welches dadurch gekennzeichnet ist, daß man

a) eine wäßrige Zusammensetzung, deren Rezeptur derjenigen der Trägerfolie entspricht, aus dem Wirkstoff sowie Stärken, Gelatinen, Glycerin und/oder Sorbit sowie gegebenenfalls natürlichen und/oder synthetischen Harzen und Gummen herstellt, und

b) diese Beschichtungsmasse kontinuierlich mittels eines Walzenauftragsverfahrens in genau vorbestimmter Menge (Schichtdicke) auf mindestens eine Seite der wasserlöslichen wirkstofffreien Folie aufbringt.

Die erfindungsgemäß hergestellte Darreichungsform weist eine Reihe wesentlicher Vorteile auf:

- Eine Trägerfolie kann für die verschiedensten Wirkstoffe verwendet werden und somit in größerer Menge wirtschaftlich produziert werden,
- die wirkstoffhaltige Schicht kann bei hochwirksamen Arzneimitteln sehr dünn sein, da das Trägermaterial die ausreichende mechanische Festigkeit gewährleistet,
- die Beschichtung haftet hervorragend auf der Trägerfolie, weil beide dieselbe Rezeptur aufweisen,
- mit Hilfe der modernen Walzen-Auftragverfahren läßt sich die wirkstoffhaltige Beschichtung mit konstanter Schichtdicke aufbringen, so daß die erforderlichen Toleranzen eingehalten werden können,
- falls eine Sterilisierung erforderlich ist, kann diese wegen der geringen Schichtdicke problemlos mittels Strahlenbehandlung erreicht werden,
- der Träger läßt sich auf der Vorder- und insbesondere der Rückseite unter Verwendung physiologisch verträglicher Druckfarben mit verschiedenen Informationen bedrucken,
- aufgrund der relativ großen Fläche von beispielsweise 4 bis 10 cm$^2$ lassen sich ausführliche Informationen für den Benutzer auf das unbeschichtete Trägermaterial oder auch nachträglich aufdrucken,
- die Dosiereinheiten lassen sich durch entsprechende Vorzerteilung, z.B. eine Perforierung, flexibel gestalten, so daß für verschiedene Dosierungen (z.B. für Erwachsene und Kinder) nur ein Produkt hergestellt werden muß; die Vorzerteilung kann ggf. auch erst in der Apotheke oder im Krankenhaus nach ärztlichen Angaben vorgenommen werden.

Mit den früher beschriebenen Darreichungsformen in Folienform hat die erfindungsgemäße darüberhinaus den Vorteil des äußerst geringen Platzbedarfes gemeinsam. Statt Faltschachteln können daher beispielsweise Taschen oder Beutel aus Kunststoffolie oder kunststoffbeschichtetem Papier verwendet werden, in welche das Produkt eingesiegelt wird, ähnlich wie feuchte Erfrischungstücher.

Die Herstellung der Trägerfolie erfolgt in an sich bekannter Weise mit einer kontinuierlich arbeitenden Folienmaschine auf Rollenbasis. Das Streichverfahren zur Herstellung der Trägerfolie arbeitet nach dem Walzenprinzip, d.h. die wasserhaltige Zusammensetzung für die Trägerfolie wird mittels Rollen und Rakel angetragen und zu dünnen Bahnen ausgestrichen, auf der Rolle vorgetrocknet und im Haupttrockengang auf die gewünschte Endfeuchte nachgetrocknet. Das erhaltene Endprodukt ist so fest und elastisch, daß es auf Rollen gewickelt werden kann und lagerfähig ist, wenn die Restfeuchtigkeit nicht zu hoch ist (Gefahr der Schimmelbildung).

Die Folienbreite kann beliebig sein und wird günstigerweise auf die Breite der Beschichtungsmaschine zugeschnitten. Es bietet sich jedoch an, bereits bei der Herstellung beide Breiten aufeinander abzustimmen.

Es ist technisch auch möglich, die Folienherstellung und die Beschichtung zeitlich nacheinander auf derselben Anlage vorzunehmen, wodurch die Wirtschaftlichkeit wesentlich erhöht werden kann.

Die verwendete Zusammensetzung wird unter Umpumpen bei der gewünschten Temperatur, Viskosität und Homogenität gehalten. Die Trocknung der Folie erfolgt anschließend in einem Wärmetunnel. Die so gewonnene Trägerfolie stellt den indifferenten Träger für die spätere Beschichtung mit verschiedene Wirkstoffe enthaltenden Beschichtungsmassen dar.

Zur Herstellung der wasserlöslichen Trägerfolie dient eine physiologisch unbedenkliche Zusammensetzung. Die "Wasserlöslichkeit" soll dabei so definiert sein, daß die Herstellung der Folie aus einer wäßrigen Zusammensetzung erfolgt und daß sich die fertige Folie später bei der Anwendung wiederum in Wasser bzw. im Magensaftmilieu löst oder darin quillt.

Als Folienbildner kommen insbesondere Gelatinen sowie Stärken (Kartoffelstärke, Weizenstärke, Maisstärke) sowie ferner Poly-N-vinylpyrrolidon (PVP), Methyl- und Ethylzellulose sowie Polyvinylalkohol (PVA) infrage. Ferner können wässerlösliche Acrylharzdispersionen Verwendung finden. Geeignete Weichmacher sind insbesondere polyfunktionelle Alkohole wie Glycerin und Sorbit (Karion®).

Die Komponenten werden in geeigneter Weise mit Wasser kalt angemischt und unter leichtem Erwärmen und ständigem Rühren zu einem streichfähigen Schleim verarbeitet. Das Einrühren von Luft muß soweit wie möglich vermieden werden, um eine klare, allenfalls leicht opaleszierende Masse zu erhalten.

Die Stärke der Trägerfolie beträgt vorzugsweise zwischen etwa 50 und 250 µm. Sie ist in weitem Maße steuerbar. Auch die Eigenschaften der Trägerfolie lassen sich durch entsprechende Kombination der Folienbildner und Weichmacher qualitativ stark beeinflussen. Die Trägerfolie soll eine möglichst gleichmäßige Stärke aufweisen (vorzugsweise z.B. 100 µm), leicht elastisch und knickfähig sein, ohne zu brechen. Dabei sollte der Stärkeanteil ausreichend hoch sein, damit beim Aufbringen der Beschichtungsmasse Feuchtigkeit aufgenommen wird, ohne daß es zu einem Kleben der Oberfläche oder zum Erweichen der ganzen Folie kommt.

Folgende Rahmenrezeptur hat sich für die Trägerfolie bewährt:

Gelatine 8 bis 10 g
Stärke 4 bis 8 g
Glycerin 1 bis 2 g

Polyvinyl-pyrrolidon 1 bis 2 g
Wasser 30 bis 50 g

Wasserlösliche natürliche und/oder synthetische Harze, z.B. Acrylharze, und Gumme sind ebenfalls geeignet. Ggf. können der Masse noch übliche weitere Stoffe zugefügt werden, z.B. Konservierungsmittel wie p-Hydroxybenzoesäure-Ester, inerte lösliche oder unlösliche Füllstoffe, Geschmackstoffe, Zucker oder andere Süßungsmittel, weitere Weichmacher, insbesondere Polyole, Wachse oder Farbstoffe.

Die Möglichkeit der vorder- und rückseitigen Bedruckung der Trägerfolie ist ein besonderer Vorteil der erfindungsgemäßen Darreichungsform. Beispielsweise können die Kennzeichnung, Angaben über die Inhaltsstoffe sowie Dosierungsangaben aufgedruckt werden. Gegebenenfalls läßt sich sogar der ganze Inhalt eines Beipackzettels rückseitig aufdrucken mit der Folge, daß ein separater Beipackzettel, der auch häufig verlorengeht, überflüssig wird. Bei Arzneimitteln, welche regelmäßig genommen werden müssen, beispielsweise bei hormonalen Contrazeptiva, kann der gesamte Verabreichungsplan so angebracht werden, daß eine einfache Einnahmekontrolle gewährleistet ist. Zur Bedruckung müssen physiologisch verträgliche Farben (Lebensmittelfarben) verwendet werden, da die Trägerfolie einen Teil der oral verabreichten Darreichungsformen bildet.

Für die wirkstoffhaltige Beschichtungsmasse findet eine wäßrige Zusammensetzung Verwendung, die physiologisch inert ist und deren Einzelkomponenten für Arzneimittel bzw. Lebensmittel geeignet sind. Wesentlich ist die gegenseitige physikalischchemische Affinität und Verträglichkeit zwischen Beschichtungsmasse und Trägerfolie, welche besonders gut ist, weil die verwendeten Komponenten gleich sind bzw. sehr ähnliche Eigenschaften besitzen. Unter Berücksichtigung des zugeführten Wirkstoffes entspricht die Rezeptur der Beschichtungsmasse demgemäß der oben für die Trägerfolie genannten, wobei die genaue Einstellung auf Feststoffgehalt und Viskosität mittels indifferenter Quell- und Füllstoffe erfolgt.

Die Masse enthält somit einmal polymere Filmbildner, vorzugsweise Gelatine und quellende oder lösliche Stärken sowie ggf. Zellulosen oder Hemizellulosen. Ferner werden Weichmacher zugesetzt, insbesondere mehrwertige Alkohole wie Glycerin oder Sorbit. Zur Einstellung der erwünschten Viskosität der Beschichtungsmasse, welche die Konsistenz eines Schleimes aufweist, finden polymere Quellstoffe Verwendung, vorzugsweise Alginate, Pectine, Chitine, Lecithine oder Polyethylenglykole. Diese letzteren Stoffe können gleichzeitig als Haftvermittler dienen. Andererseits können auch wasserlösliche synthetische oder natürliche Harze oder Gumme oder Gummi arabicum zugesetzt werden, um die Haftung der Beschichtung auf dem Trägermaterial zu verbessern. Schließlich können noch Konservierungsmittel wie z.B. p-Hydroxybenzoesäureester, Farbstoffe (Lebensmittelfarbstoffe), Pigmente wie Titandioxid oder Aroma- und Süßstoffe zugesetzt werden.

Coatingmassen mit einem Wassergehalt von ungefähr 50% und einer Viskosität von etwa 30 bis zu 10 000 cPs haben sich als besonders geeignet erwiesen. Die Rezeptur und Herstellung ähnelt derjenigen eines Arzneimittelsaftes, in welchem der Wirkstoff bzw. die Wirkstoffkombination gelöst oder gleichmäßig dispergiert wird. Die Beschichtungsmasse muß ausreichende Homogenität und galenische Stabilität aufweisen, damit ein gleichmäßiger Wirkstoffgehalt der fertigen Beschichtung sichergestellt ist.

In dieser Grundmasse wird der Wirkstoff gelöst bzw. dispergiert. Im Fall der Verwendung einer Dispersion muß der Wirkstoff für eine gleichmäßige Verteilung äußerst feinteilig sein. Vorzugsweise liegt die mittlere Teilchengröße im Bereich von etwa 1 bis 20 μm.

Die gewünschte Dosis des Wirkstoffes und die angestrebte Fläche der Dosiseinheiten bestimmen letztlich die Dicke der Schicht, wobei der Feuchtigkeitsgehalt der Beschichtungsmasse und der fertigen Beschichtung zu berücksichtigen sind.

Die erfindungsgemäße Darreichungsform ist besonders geeignet für Arzneimittel, welche niedrig dosiert verabreicht werden, d.h. bei welchen die Einzeldosis für die orale Applikation zwischen 0 mg (Placebo) und etwa 20 mg liegt. Geeignete Arzneimittelwirkstoffe finden sich in allen Bereichen der oralen Therapie; hervorzuheben sind u.a. Analeptika, Antibiotika, Antidiabetika, Antiemetika, Antiepileptika, Antihypertonika, Cortikoide, Geriatrika, Hypnotika, Cardiaka, Hypostatika und Biowirkstoffe.

In einem Beschichtungsgang lassen sich ca. 4 bis 20 g Wirkstoff je m² (= 10.000 cm²) Trägerfolie aufbringen, so daß 10 cm² (= 2 übliche Briefmarken) bis zu 20 mg Wirkstoff aufnehmen können.

Die Beschichtungsmasse wird normalerweise auf eine Seite der Trägerfolie aufgebracht, doch ist auch eine beidseitige Beschichtung, insbesondere bei zwei verschiedenen Wirkstoffen möglich. Jede Beschichtung kann einen oder mehrere Arzneimittelwirkstoffe enthalten. Falls bei Verwendung mehrerer Wirkstoffe diese nicht ohne weiteres miteinander verträglich sind und in einer Beschichtungsmasse enthalten sein können, ist es bei der erfindungsgemäßen Darreichungsform möglich, die Beschichtung in mehreren Schichten unterschiedlicher Zusammensetzung aufzubringen und die Wirkstoffe dadurch voneinander zu trennen, wobei erforderlichenfalls eine wirkstofffreie Zwischenschicht vorgesehen werden kann. Weiterhin ist es möglich, über der wirkstoffhaltigen Schicht noch eine weitere Schutzschicht vorzusehen, welche den/die Wirkstoff(e) genen eine Berührung mit der Atmosphäre und/oder gegen Licht schützt. In diesen Fällen muß die Schutzschicht demgemäß luft- und feuchtigkeitsundurchlässig und/oder durch Zusatz entsprechender Farbstoffe bzw. Pigmente lichtundurchlässig sein.

Weiterhin kann durch entsprechenden Aufbau der Beschichtung die Wirkstoffabgabe nach Verabreichung des Arzneimittels gesteuert werden. Beispielsweise ist es möglich, eine Wirkstoffschicht zwischen mindestens zwei weiteren Schichten anzuordnen, welche die Wirkstoffresorption im Magen/Darmtrakt in an sich bekannter Weise steuern.

Dabei kann die Wirkstoffschicht z.B. zwischen zwei säureunlöslichen Schichten angeordnet werden, so daß bei Verabreichung der Magen passiert wird und die Resorption erst im Darmtrakt erfolgt. In ähnlicher Weise können unterschiedliche Wirkstoffe in verschiedenen Schichten übereinander auf die Trägerfolie aufgebracht werden, damit die Resorption nacheinander und/oder verzögert erfolgt.

Ähnliche pharmakokinetische Effekte lassen sich durch das Einarbeiten (z.B. Suspendieren) von unterschiedlich vorbehandelten mikroverkapselten Wirkstoffen erzielen.

Die Beschichtung des Trägermaterials mit der wirkstoffhaltigen Beschichtungsmasse erfolgt mittels eines Walzenauftragverfahrens. Dieses für die quantitative Beschichtung besonders geeignete Verfahren arbeitet nach einem dem Tiefdruck ähnlichen Verfahren, welches als "Akkugravur" bezeichnet wird. Hierfür geeignete Maschinen sind im Handel (Fa. Pagendarm, Hamburg) und erlauben Auftragsgewichte bis zu 80 g/m² bei Bahngeschwindigkeiten von mehreren 100 m/min. Die reproduzierbare Gewichtskonstanz liegt für 20 g/m² bei nur +/- 2,5% für 1 g/m² und für ca. +/- 10% über die gesamte Fläche. Der Auftrag der Beschichtungsmasse erfolgt kontinuierlich über Walzen mit spezieller Feingravur, wobei die eingravierten Rillen zur Laufrichtung der Trägerfolie vorzugsweise einen Winkel von 30 bis 60, insbesondere 45° bilden. In die Walzen können 27 bis 80 Rillen/cm eingeätzt sein. Entsprechend ihrer Form und Tiefe kann die Gravur eine definierte Menge der Beschichtungsmasse aufnehmen und anschließend an die Trägerfolie weitergeben. Durch Variation der Vorlaufgeschwindigkeit, der Laufrichtung und der Gravur sowie durch indirektes Auftragen über eine weitere geschwindigkeitsvariable Walze lassen sich die Beschichtungsmengen sehr exakt einstellen.

Eine zweiseitige Beschichtung ergibt häufig Vorteile, da Probleme durch Verwerfen des Trägermaterials und durch unterschiedliche Hygroskopizität ausgeglichen werden. Mehrfach- und auch Streifenbeschichtungen, ja sogar Druckbildbeschichtungen, sind möglich und bieten bei der Verarbeitung von inkompatiblen Wirkstoffen eine große Variabilität.

Ein anderes geeignetes Auftragverfahren entspricht dem Streichen von Papier oder von Folien. Dabei werden Rohpapiere dadurch verbessert, daß sie ein- oder zweiseitig mit Coatingmaterialien beschichtet werden. Die wässrigen Beschichtungsmassen gelangen zunächst auf ein Walzwerk, welches sie mittels einer rotierenden Walze aufnimmt, mit einen Rakel bestimmten Abstandes auf eine definierte Schichtdicke abstreift, worauf die Walze die Beschichtungsmasse auf den Träger abgibt. Die Trägerfolie, welche 0,30 bis 7,50 m breit sein kann, durchläuft anschließend einen Trockentunnel und wird dann auf Rollen aufgewickelt. Dieser Vorgang ist in einem oder mehreren Schritten ein- oder zweiseitig wiederholbar, wobei auch eine bereits beschichtete Fläche nochmals beschichtet werden kann. Das Gewicht des Trägermaterials nimmt um das der Trockenmasse zu. Die Genauigkeit des Auftragverfahrens mittels dieses Rakel-Verfahrens liegt reproduzierbar bei +/- 5%. Sie ist abhängig von der jeweiligen Schichtdicke, die variabel zwischen 4 und 40 g/m² betragen kann. Innerhalb der einzelnen Fertigungen kann eine Gewichtstoleranz pro Flächeneinheit bis unter +/- 1 % erreicht werden.

Bei Aufbringung mehrerer Schichten, wie dies oben bereits beschrieben wurde, werden diese nacheinander aufgebracht, wobei ggf. jede Beschichtung zuvor eine Trocknungsstation durchläuft. Diese kann beispielsweise aus einem temperierten Walzenpaar und einem in Sektionen steuerbaren Trockentunnel bestehen. Nach dem letzten Beschichtungsvorgang wird das beschichte Material auf Rollen aufgewickelt.

Die wirkstoffbeschichtete Trägerfolie wird anschließend in Dosiseinheiten vorzerteilt, welche ähnlich wie Briefmarken abtrennbar sind. Normalerweise wird diese Vorzerteilung beim Arzneimittelhersteller erfolgen, es ist jedoch auch denkbar, das beschichtete Material beispeilsweise an Krankenhäuser oder Apotheken auszuliefern, wo dann die Vorzerteilung dosisabhängig oder auch individuell nach ärztlicher Vorgabe durchgeführt werden kann.

Die Vorzerteilung erfolgt in besonders einfacher Weise durch Perforierung oder Stanzung, wobei es möglich ist, diesen Schritt mit der Bedruckung des Trägermaterials zu kombinieren. In vielen Fällen wird es allerdings günstiger sein, die Bedruckung des Trägermaterials vor der Beschichtung vorzunehmen.

Vor oder besser nach Vorzerteilung der wirkstoffhaltigen Beschichtung in Dosiseinheiten wird das beschichtete Trägermaterial zu gebrauchsfertigen Abschnitten zerschnitten, welche eine bestimmte Anzahl von Dosiseinheiten enthalten. Es ist auch denkbar, das Material auf Rollen zu schmalen Bändern zu zerschneiden. Von einer solchen Einzelrolle können dann die einzelnen Dosiseinheiten ähnlich wie einzelne Briefmarken abgetrennt werden.

Da als Grundstoffe für die Herstellung der erfindungsgemäßen Darreichungsform überwiegend Naturstoffe wie Stärken und Gelatine verwendet werden, erhält man insgesamt Produkte, welche den bekannten Oblaten ähneln und deren orale Einnahme keinerlei Schwierigkeiten bereitet. Wichtig ist, daß das Fertigprodukt weitgehend von Wasser befreit ist, d.h. einen Wassergehalt von wengier als 10 und vorzugsweise von weniger als 2% aufweist, da sonst Schimmelbildung auftreten kann.

Vorstehend wurde die Erfindung im wesentlichen im Zusammenhang mit Arzneimitteln beschrieben, worauf sie jedoch keineswegs beschränkt ist. Beispielsweise lassen sich in derselben Weise auch Dosierungsformen für chemische Reagentien, Aromastoffe und dergleichen herstellen.

Zur näheren Erläuterung der Erfindung soll das nachfolgende Ausführungsbeispiele dienen.

Beispiel

Herstellung einer Arzneimittel-Darreichungsform in Form einer beschichteten Folie.

Zur Herstellung einer wasserlöslichen Trägerfolie wurde von folgender Zusammensetzung ausgegangen:

Gelatine 10,0 Gew.-Teile = 25%
Kartoffelstärke 8,0 Gew.-Teile = 20%
Glycerin 1,5 Gew.-Teile = 3,75%
gereinigtes Wasser 20,5 Gew.-Teile = 51,25%

Die Viskosität der schleimartigen Zusammensetzung betrug bei 50°C ca. 3000 cPs. Mit Hilfe des Streichverfahrens wurde die Masse zu einer Folie verarbeitet, welche nach dem Trocknen noch 9,3% Restwasser enthielt.

Unter Verwendung derselben Grundstoffe wie für die Trägerfolie wurde die Beschichtungsmasse gemäß folgender Rezeptur hergestellt:

Gelatine 10,0 Gew.-Teile = 18,2%
Kartoffelstärke 5,0 Gew.-Teile = 9,1%
Glycerin 1,0 Gew.-Teile = 1,8%
Wirkstoff 5,0 Gew.-Teile = 9,1%
gereinigtes Wasser 34,0 Gew.-Teile = 61,8%

Die Viskosität der schleimartigen Zusammensetzung betrug temperatur- und wirkstoffabhängig zwischen 4.000 und 10.000 cPs. Zur Herstellung der Beschichtungsmasse wurde zunächst die Gelatine in einer ausreichenden Menge Wasser gelöst. Dazu wurde Wasser von 90 bis 95°C vorgelegt, in das die Gelatine unter Rühren eingetragen wurde. In einem getrennten Ansatz wurde der Wirkstoff zusammen mit dem Glycerin in Wasser gelöst. Schließlich wurde die Kartoffelstärke bei 50 bis 60°C unter Rühren in einer ausreichenden Menge Wasser angerührt. Die Gelatinelösung und die Kartoffelstärkesuspension wurden zusammengegeben und die Wirkstoffsuspension wurde in die Mischung langsam eingerührt, wobei Lufteinschlüsse vermieden wurden. Die Temperatur wurde auf 55 bis 60°C gehalten. Zuletzt wurde der gewünschte Wassergehalt durch Zugabe von weiterem Wasser eingestellt.

Die Beschichtungsmasse wurde mittels Akkugravur mit einem Naßbeschichtungsgewicht von 55 g/m² auf die Trägerfolie aufgebracht. Nach dem Trocknen betrug das Beschichtungsgewicht 23 g/m² entsprechend einem Wirkstoffgehalt von 5 g/m². Die wirkstoffbeschichtete Folie wurde anschließend kastenartig perforiert, so daß die einzelnen Abschnitte bei Abmessungen von 2 x 2,5 cm eine Fläche von 5 cm² aufwiesen. Ein solcher Abschnitt enthielt 2.5 mg Wirkstoff.

Nach dem Trocknen lag die Restfeuchtigkeit des Produktes bei 8,6%.

Es wurde eine Darreichungsform erhalten, welche bei oraler Einnahme im Mund rasch quillt und zergeht und sich demgemäß leicht schlucken läßt.

## Patentansprüche

1. Verfahren zur Herstellung einer Darreichungs- und Dosierungsform für Arzneimittelwirkstoffe, Reagentien oder andere Wirkstoffe in Form einer wasserlöslichen Folie auf Basis von Stärken, Gelatinen, Glycerin und/oder Sorbit sowie gegebenenfalls natürlichen und/oder synthetischen Harzen und Gummen, dadurch gekennzeichnet daß man
   a) eine wässrige Zusammensetzung, deren Rezeptur derjenigen der Trägerfolie entspricht, aus dem Wirkstoff sowie Stärken, Gelatinen, Glycerin und/oder Sorbit sowie gegebenenfalls natürlichen und/oder synthetischen Harzen und Gummen herstellt, und
   b) diese Beschichtungsmasse kontinuierlich mittels eines Walzenauftragsverfahrens in genau vorbestimmter Menge (Schichtdicke) auf mindestens eine Seite der wasserlöslichen wirkstofffreien Folie aufbringt.

2. Verfahren nach Anspruch 1, dadurch gekennzeichnet, daß man der Zusammensetzung für die Trägerfolie und die Beschichtung zusätzlich inerte lösliche und/oder unlösliche Füllstoffe, Zucker und/oder andere Süßungsmittel, weitere Weichmacher, insbesondere Polyole, Wachse, Farbstoffe, Geschmacksstoffe und/oder Konservierungsmittel zusetzt.

3. Verfahren nach einem der Ansprüche 1 oder 2, dadurch gekennzeichnet, daß man für die Herstellung der Trägerfolie und der Beschichtungsmasse eine Zusammensetzung verwendet, die 8 bis 10 Gew.-Teile Gelatine, 4 bis 8 Gew.-Teile Stärke, 1 bis 2 Gew.-Teile Glycerin und 20 bis 50 Gew.Teile Wasser enthält.

4. Verfahren nach Anspruch 3, dadurch gekennzeichnet, daß man eine Beschichtungsmasse einsetzt, die bis zu 10 Gew.-Teile des Wirkstoffes enthält.

5. Verfahren nach einem der Ansprüche 1 bis 4, dadurch gekennzeichnet, daß man der Beschichtungsmasse zur Einstellung der Viskosität indifferente Quell- und Füllstoffe zusetzt.

6. Verfahren nach einem der Ansprüche 1 bis 5, dadurch gekennzeichnet, daß man die Beschichtungsmasse kontinuierlich mittels Rasterwalzen, welche eine genau definierte Menge der Beschichtungsmasse aufnehmen und wieder abgeben, auf die Trägerfolie aufbringt.

7. Verfahren nach einem der Ansprüche 1 bis 5, dadurch gekennzeichnet, daß man die Beschichtungsmasse kontinuierlich mittels glatter Walzenpaare, welche in geschwindigkeitsversetztem Gleichlauf die Masse aufnehmen und in definierter Menge abgeben, auf die Trägerfolie aufbringt.

8. Verfahren nach einem der Ansprüche 1 bis 7, dadurch gekennzeichnet, daß man zur Herstellung eines Kombinationspräparates auf die Ober- und die Unterseite der Trägerfolie unterschiedliche Wirkstoffe aufbringt.

## Claims

1. Process for the manufacture of a presentation and dosage form for pharmaceutical active substances, reagents or other active substances in the form of a water-soluble foil based on starches, gelatines, glycerin and/or sorbite and also in some cases on natural and/or synthetic resins and gums, characterized in that
a) an aqueous composition, the formulation of which corresponds to that of the carrier foil, is manufactured from the active substance and from starches, gelatines, glycerin and/or sorbite and also in some cases from natural and/or synthetic resins and gums, and that

b) this coating substance is applied continuously in a precise pre-determined quantity (layer thickness) to at least one side of the active-substance-free-water-soluble foil by means of a roller coating process.

2. Process according to claim 1, characterized in that inert, soluble and/or insoluble fillers, sugars and/or other sweeteners, other softeners, particularly polyols, waxes, colorants, flavouring agents and/or preservatives are also added to the composition for the carrier foil and the coating.

3. Process according to one of claims 1 or 2, characterized in that, for the manufacture of the carrier foil and the coating substance, a composition is used which contains 8 to 10 parts by weight of gelatine, 4 to 8 parts by weight of starch, 1 to 2 parts by weight of glycerin and 20 to 50 parts by weight of water.

4. Process according to claim 3, characterized in that a coating substance is used which contains up to 10 parts by weight of the active substance.

5. Process according to one of claims 1 to 4, characterized in that inert swelling agents and fillers are added to the coating substance to regulate the viscosity.

6. Process according to one of claims 1 to 5, characterized in that the coating substance is continuously applied by means of grid rollers which take up and then release a precisely defined quantity of the coating substance.

7. Process according to one of claims 1 to 5, characterized in that the coating substance is applied to the carrier foil continuously by means of smooth pairs of rollers synchronized but out of phase which take up the substance and release a pre-defined quantity.

8. Process according to one of claims 1 to 7, characterized in that different active substances are applied to the top and bottom of the carrier foil for the manufacture of a compound preparation.

**Revendications**

1. Procédé de fabrication d'une forme d'administration et de dosage pour des principes actifs de médicaments, des réactifs ou d'autres substances actives, sous forme d'une feuille hydrosoluble à base d'amidons, de gélatines, de glycérol et/ou de sorbitol, et éventuellement de résines et gommes naturelles et/ou synthétiques, procédé caractérisé en ce que l'on

a) fabrique une composition aqueuse, dont la formulation correspond à celle de la feuille support, à partir de la substance active ainsi que d'amidons, de gélatines, de glycérol et/ou de sorbitol, et éventuellement de résines et gommes naturelles et/ou synthétiques, et

b) dépose en continu, à l'aide d'un cylindre d'enduction, cette masse, en quantité exactement prédéterminée (épaisseur de couche), sur au moins une des faces de la feuille hydrosoluble dépourvue de substance active.

2. Procédé selon la revendication 1, caractérisé en ce que l'on ajoute en plus, à la composition pour la feuille support et le revêtement, des charges inertes solubles et/ou insolubles, des sucres et/ou d'autres édulcorants, en outre des plastifiants, en particulier des polyols, des cires, des colorants, des aromatisants et/ou des conservateurs.

3. Procédé selon l'une des revendications 1 ou 2, caractérisé en ce que, pour la fabrication de la feuille support et du revêtement, on utilise une composition qui renferme de 8 à 10 parties en poids de gélatine, 4 à 8 parties en poids d'amidon, 1 à 2 parties en poids de glycérol et 20 à 50 parties en poids d'eau.

4. Procédé selon la revendication 3, caractérisé en ce que l'on met en œuvre une masse d'enduction qui renferme jusqu'à 10 parties en poids de la substance active.

5. Procédé selon l'une des revendications 1 à 4, caractérisé en ce que l'on ajoute des agents gonflants et charges inertes à la masse d'enduction, pour ajuster la viscosité.

6. Procédé selon l'une des revendications 1 à 5, caractérisé en ce que l'on dépose en continu la masse d'enduction sur la feuille support, à l'aide de cylindres à trame, qui prennent puis rétrocèdent une quantité exactement définie de la masse d'enduction.

7. Procédé selon l'une des revendications 1 à 5, caractérisé en ce que l'on dépose en continu la masse d'enduction sur la feuille support, à l'aide de paires de cylindres lisses, qui prennent la masse avec un syndrome décalé de la vitesse et la rétrocèdent en quantité définie.

8. Procédé selon l'une des revendications 1 à 7, caractérisé en ce que, pour fabriquer une préparation combinée, on dépose différentes substances actives sur la face supérieure et sur la face inférieure de la feuille support.